(19) **Europäisches Patentamt / European Patent Office / Office européen des brevets**

(11) **EP 3 628 304 B1**

(12) **EUROPEAN PATENT SPECIFICATION**

(45) Date of publication and mention
of the grant of the patent:
**14.07.2021 Bulletin 2021/28**

(51) Int Cl.:
*A61K 8/34* (2006.01)       *A61K 8/92* (2006.01)
*A61K 8/04* (2006.01)       *A61K 8/14* (2006.01)
*A61K 8/02* (2006.01)       *A61Q 5/12* (2006.01)
*A61Q 19/00* (2006.01)     *A61K 8/86* (2006.01)

(21) Application number: **18196989.0**

(22) Date of filing: **26.09.2018**

(54) **COSMETIC GEL COMPRISING A TRIGLYCERIDE OIL**

KOSMETISCHES GEL MIT EINEM TRIGLYCERIDÖL

GEL COSMÉTIQUE COMPRENANT UNE HUILE DE TRIGLYCÉRIDE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**01.04.2020 Bulletin 2020/14**

(73) Proprietor: **Clariant International Ltd**
**4132 Muttenz (CH)**

(72) Inventors:
• **Dahms, Gerd**
**47138 Duisburg (DE)**

• **Wagdare, Nagesh Appasaheb**
**Navi**
**Mumbai 410210 (IN)**

(74) Representative: **Kampen, Daniela**
**Clariant Produkte (Deutschland) GmbH**
**Patent & License Management Chemicals**
**Industriepark Höchst / G 860**
**65926 Frankfurt am Main (DE)**

(56) References cited:
WO-A1-2010/052093       KR-B1- 100 712 255
US-A1- 2001 047 039       US-A1- 2013 201 785

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

FIELD OF THE INVENTION

The present invention relates to a cosmetic gel, a cosmetic product comprising a packaged cosmetic gel, uses of the cosmetic gel and a process for making the cosmetic gel.

BACKGROUND OF THE INVENTION

Triglyceride oil, such as coconut oil and palm kernel oil, is rapidly growing in popularity for a wide variety of uses and benefits. Triglyceride oil finds frequent application in the cosmetic, skin and hair care fields. An especially popular application of triglyceride oil is for hair.

A cleansing oil gel is known from KR 10-0712255. The gel is provided using a combination of hydrogenated lecithin, polyglycerin fatty acid ester, oil, polyol and water. It is desirable to provide a gel using fewer components. In addition, it is desirable to provide a gel with improved stability. In addition, it can be challenging to deposit triglyceride oil effectively on hair and skin and more effective methods are also desirable.

US2001047039 A1 discloses a skin moisturiser comprising a liquid crystal gel network comprising multilamellar vesicles. Example 1, composition 1 shows a composition comprising a surfactant component comprising a vesicle-forming surfactant having an HLB more than 6 (distearyldimonium chloride), an oily phase comprising a triglyceride oil and an aqueous phase comprising a polyol.

It is with this background that the present invention has been devised.

SUMMARY OF THE INVENTION

In a first aspect, the present invention relates to a cosmetic gel comprising multilamellar vesicles dispersed in an aqueous phase, wherein the multilamellar vesicles have the shape of a rotational body comprising two or more concentric lipid double layers, the cosmetic gel comprising:

a) a surfactant component, comprising at least one vesicle-forming surfactant, which is a surfactant having a HLB value of greater than 6;
b) an oily phase comprising a triglyceride oil;
c) an aqueous phase comprising a water-soluble polyhydric alcohol;

and wherein the multilamellar vesicles have a median particle size d(50) from 50 to 800 nanometres and wherein the particle size distribution is Gaussian and the standard deviation is less than 30% of the median particle size d(50), and wherein the cosmetic gel comprises from 15 wt.% to 80 wt.% of triglyceride oil.

DETAILED DESCRIPTION OF THE INVENTION

In this document, including in all embodiments of all aspects of the present invention, the following definitions apply unless specifically stated otherwise. All percentages are by weight (w/w) of the total composition. All ratios are weight ratios. "wt.%" means percentage by weight. References to 'parts' e.g. a mixture of 1 part X and 3 parts Y, is a ratio by weight. "QS" or "QSP" means sufficient quantity for 100% or for 100g. +/- indicates the standard deviation. All ranges are inclusive and combinable. The number of significant digits conveys neither a limitation on the indicated amounts nor on the accuracy of the measurements. All numerical amounts are understood to be modified by the word "about". All measurements are understood to be made at 23°C and at ambient conditions, where "ambient conditions" means at 1 atmosphere (atm) of pressure and at 50% relative humidity. "Relative humidity" refers to the ratio (stated as a percent) of the moisture content of air compared to the saturated moisture level at the same temperature and pressure. Relative humidity can be measured with a hygrometer, in particular with a probe hygrometer from VWR® International. Herein "min" means "minute" or "minutes". Herein "mol" means mole. Herein "g" following a number means "gram" or "grams". "Ex." means "example". All amounts as they pertain to listed ingredients are based on the active level ('solids') and do not include carriers or by-products that may be included in commercially available materials. Herein, "comprising" means that other steps and other ingredients can be in addition. The compositions, formulations, methods, uses, kits, and processes of the present invention can comprise, consist of, and consist essentially of the elements and limitations of the invention described herein, as well as any of the additional or optional ingredients, components, steps, or limitations described herein. Embodiments and aspects described herein may comprise or be combinable with elements, features or components of other embodiments and/or aspects despite not being expressly exemplified in combination, unless an incompatibility is stated. "In at least one embodiment" means that one or more embodiments, optionally all embodiments or a large subset of embodiments, of the present invention has/have the subsequently described feature. Where amount ranges are given, these are to be understood as being the total amount of said ingredient in the composition, or where more than one species fall within the scope of the ingredient definition, the total amount of all ingredients fitting that definition, in the composition. For example, if the composition comprises from 1% to 5% fatty alcohol, then a composition comprising 2% stearyl alcohol and 1% cetyl alcohol and no other fatty alcohol, would fall within this scope.

"Molecular weight" or "M.Wt." or "MW" and grammatical equivalents mean the number average molecular weight.

"Viscosity" is measured at 25°C using a HAAKE Rotation Viscometer VT 550 with cooling/heating vessel and sensor systems according to DIN 53019 at a shear rate of 12.9 S-1.

"Water-soluble" refers to any material that is sufficiently soluble in water to form a clear solution to the naked eye at a concentration of 0.1 % by weight of the material in water at 25°C. The term "water-insoluble" refers to any material that is not "water-soluble".

"Dry" or "substantially dry" means comprising less than 5%, less than 3% or, less than 2%, less than 1%, or about 0% of any compound or composition being in liquid form when measured at 25°C at ambient conditions. Such compounds or compositions being in liquid form include water, oils, organic solvents and other wetting agents. "Anhydrous" means that the composition comprises less than 5%, less than 3% or, less than 2%, less than 1%, or about 0% water by total weight of the composition.

"Substantially free from" or "substantially free of" means less than 1%, or less than 0.8%, or less than 0.5%, or less than 0.3%, or about 0%, by total weight of the composition or formulation.

"Hair" means mammalian keratin fibres including scalp hair, facial hair and body hair. It includes such hair still being attached to a living subject and also hair that has been removed therefrom such as hair swatches and hair on a doll/mannequin. In at least one embodiment, "hair" means human hair. "Hair shaft" or "hair fibre" means an individual hair strand and may be used interchangeably with the term "hair."

"Cosmetically acceptable" means that the compositions, formulations or components described are suitable for use in contact with human keratinous tissue without undue toxicity, incompatibility, instability, allergic response, and the like. All compositions and formulations described herein which have the purpose of being directly applied to keratinous tissue are limited to those being cosmetically acceptable.

"Derivatives" includes but is not limited to, amide, ether, ester, amino, carboxyl, acetyl, acid, salt and/or alcohol derivatives of a given compound. In at least one embodiment, "derivatives thereof' means the amide, ether, ester, amino, carboxyl, acetyl, acid, salt and alcohol derivatives.

"Monomer" means a discrete, non-polymerised chemical moiety capable of undergoing polymerisation in the presence of an initiator or any suitable reaction that creates a macromolecule e.g. such as polycondensation, polyaddition, anionic or cationic polymerization. "Unit" means a monomer that has already been polymerised i.e. is part of a polymer.

"Polymer" means a chemical formed from the polymerisation of two or more monomers. The term "polymer" shall include all materials made by the polymerisation of monomers as well as natural polymers. Polymers made from only one type of monomer are called homopolymers. Herein, a polymer comprises at least two monomers. Polymers made from two or more different types of monomers are called copolymers. The distribution of the different monomers can be random, alternating or block-wise (i.e. block copolymer). The term "polymer" used herein includes any type of polymer including homopolymers and copolymers.

"Kit" means a package comprising a plurality of components. "Kit" may be referred to as "kit-of-parts". An example of a kit is, for example, a first composition and a separately packaged second composition and optionally application instructions.

The triglyceride oil according to the present invention consists of one or more triglyceride oils. The or each triglyceride oil may comprise one or more hydrocarbon chains having from 8 to 12 carbon atoms. In at least one embodiment, the or each triglyceride oil comprises saturated or (poly)unsaturated hydrocarbon chains.

The triglyceride oil may comprise coconut oil, palm kernel oil, palm stearin oil, cotton stearin oil, corn oil, soybean oil, rice oil, jojoba oil, babusscu oil, pumpkin oil, grapeseed oil, sesame oil, walnut oil, apricot oil, macadamia oil, avocado oil, sweet almond oil, lady's-smock oil, castor oil, olive oil, peanut oil, rapeseed oil, argan oil, abyssinian oil, mustard oil, sesame oil, sunflower oil, olive oil, linseed oil, neem oil, almond oil, gooseberry oil, jojoba oil, rapeseed oil, Jasmine oil, caprylic capric triglyceride or mixtures thereof.

It has been found that triglyceride oils may be encapsulated in multi-lamellar vesicles comprising two or more concentric lipid double layers. These multilamellar vesicles may be manufactured to form sub-micron sized particles, which may be provided with a narrow particle size distribution. The small particle size may allow more effective application of the triglyceride oil to skin or hair than larger particles. The narrow particle size distribution may avoid coalescence of the multilamellar vesicles and ensure gel stability. The gel form provides a carrier for the sub-micron sized, dispersed multilamellar vesicle particles and may be more pleasant for a user to apply than an oily product alone. Once applied to skin or hair, the cosmetic gel may form a film providing benefits such as gloss, shine and non-greasiness.

The inclusion of some triglyceride oils in cosmetic compositions may be challenging, because they are solid at ambient temperatures. In consequence, these triglyceride oils must be heated above their melting temperatures prior to use. Warming such compositions in the microwave or directly over heat in a pan may degrade the quality of the oil, making it less beneficial. This process of warming and the application of the warmed composition to skin or hair may also be hazardous to the user. Triglyceride oils which may be solid at ambient temperature include coconut oil, palm kernel oil, palm stearin oil and cotton stearin oil. The inventors have surprisingly discovered that triglyceride oil encapsulated in multi-lamellar vesicles according to the invention remains in a liquid state, even if a triglyceride oil, such as coconut oil, is present which, on its own, would be solid at the ambient temperature. The cosmetic gel according to the invention therefore has the advantage that application of heat prior to application is unnecessary, saving work and energy and avoiding a potentially hazardous heating step.

The triglyceride oil may comprise one or more triglyceride oils which have a melting point which is greater than or equal to 20 degrees Celsius, preferably greater than or equal to 24 degrees Celsius. In one embodiment, the triglyceride oil consists of one or more triglyceride oils, each of which has a melting point which is greater than or equal to 20 degrees Celsius, preferably greater than or equal to 24 degrees Celsius.

Advantageously, the triglyceride oil comprises and preferably consists of coconut oil.

The composition of the invention is in the form of a cosmetic gel. In at least one embodiment, the gel is temperature stable within the range of 20°C to 40°C.

The multilamellar vesicles of the present invention have a shape of a rotational body, such as that of a sphere or an ellipsoid or that of a disk or other shape of a solid of revolution.

The median particle size d(50) of the vesicles of the present invention is between 50 and 800 nm, preferably between 100 and 500 nm and more preferably between 100 and 200 nm.

The median particle size d(50) is determined by laser diffraction analysis, for example by using a Horiba LA 940 or Mastersizer 3000 from Malvern using the "Mie Scattering Theory" evaluation. Median values are defined as the value where half of the population resides above this point, and half resides below this point. For particle size distributions the median is called the "d(50)" or "D50".

The particle size distribution of the multilamellar vesicles is found to be Gaussian. The multilamellar vesicles may be manufactured so that and the standard deviation is less than 30%, preferably less than 20% and more preferably less than 10% of the median particle size d(50). The narrow particle size distribution may avoid coalescence of the multilamellar vesicles and ensure gel stability.

In case of vesicles having axes of different length, such as vesicles having the shape of an ellipsoid or of a disk, the largest axis determines the mean diameter.

According to the invention, the multilamellar vesicles comprise at least one surfactant having an HLB value of greater than 6 and a triglyceride oil. These surfactants are able to form lyotropic lamellar liquid-crystalline phases. The formation of liquid-crystalline structures is dependent upon the geometries of the surfactant and the triglyceride oil, which can be expressed by the packing parameter PP.

$$PP = V_0 / (a_e * l_0)$$

$V_0$     surfactant tail volume
$A_e$     equilibrium area per molecule at the aggregate interface
$l_0$     tail length

The cosmetic gel comprises an oily phase which comprises at least the triglyceride oil. In at least one embodiment, the oily phase consists of triglyceride oil.

In at least one embodiment, the oily phase may comprise one or more further oily substances, which are not triglyceride oils.

In at least one embodiment, the formulation comprises a further oily substance selected from the group consisting of perfume oils, oily vitamins, such as vitamin E and its derivatives, silicone oils, volatile or nonvolatile, linear, branched or cyclic, optionally with organic modification; phenylsilicones; silicone resins and silicone gums; mineral oils such as paraffin oil or vaseline oil; oils of animal origin such as perhydrosqualene, lanolin; synthetic oils such as purcellin oil, isoparaffins, linear and/or branched fatty alcohols and fatty acid esters, preferably guerbet alcohols having 6 to 18, preferably 8 to 10, carbon atoms; esters of linear ($C_6$-$C_{13}$) fatty acids with linear ($C_6$-$C_{20}$) fatty alcohols; esters of branched ($C_6$-$C_{13}$) carboxylic acids with linear ($C_6$-$C_{20}$) fatty alcohols, esters of linear ($C_6$-$C_{18}$) fatty acids with branched alcohols, especially 2-ethylhexanol; esters of linear and/or branched fatty acids with polyhydric alcohols (such as dimerdiol or trimerdiol, for example) and/or guerbet alcohols; esters such as dioctyl adipate, diisopropyl dimer dilinoleate; propylene glycols/dicaprylate or waxes such as beeswax, paraffin wax or microwaxes, alone or in combination with hydrophilic waxes, such as cetylstearyl alcohol, for example; fluorinated and perfluorinated oils; fluorinated silicone oils; mixtures of the aforementioned compounds.

In at least one embodiment, the cosmetic gel comprises at least 35 wt.%, or from 36 wt.% to 85 wt.%, preferably from 38 wt.% to 80 wt.%, more preferably 40 wt.% to 75 wt.%, even more preferably 50 wt.% to 75 wt.% oily phase.

The nature of a surfactant can be represented by the hydrophilic-lipophilic balance (HLB) of the molecule. The degree of this hydrophilic-lipophilic balance can be determined by calculating values for the different regions of the molecule, as described by Griffin in 1949 and 1954. Griffin's method has been primarily developed for non-ionic surfactants as described in 1954 works as follows

$$HLB = 10*M_h/M$$

where

$M_h$ is the molecular mass of the hydrophilic portion of the molecule, and M is the molecular mass of the whole molecule, giving a result on a scale of 0 to 20. An HLB value of 0 corresponds to a completely lipophilic molecule, and a value of 20 corresponds to a completely hydrophilic molecule.

The term "HLB" as used in this specification for nonionic surfactants is calculated by the above formula. The method of Griffin is published, for example, in Journal of the Society of Cosmetic Chemists, 5 (4), 249-256 (1954). The term "HLB" as used in this specification for anionic, cationic or amphoteric surfactants is calculated by the method of Davies. This method is published, for example, in Gas/Liquid and Liquid/Liquid Interfaces. Proceedings of 2nd International Congress Surface Activity, pp. 426-438, Butterworths, London 1957.

The cosmetic gel according to the invention comprises a surfactant component. The surfactant component comprises at least one vesicle-forming surfactant. A vesicle-forming surfactant is a surfactant having a HLB value of greater than 6. In combination with other components, such as the triglyceride oil, such a surfactant may form multilamellar vesicles according to the invention.

In at least one embodiment, the cosmetic gel according to the invention comprises no hydrogenated lecithin.

In at least one embodiment, the cosmetic gel comprises from 1 wt.% to 40 wt.% vesicle-forming surfactant. In at least one embodiment, the cosmetic gel comprises from 1.5 wt.% to 20 wt.%, preferably from 2 wt.% to 15 wt.%, more preferably from 2.5 wt.% to 12 wt.%, even more preferably from 3 wt.% to 10 wt.% of vesicle-forming surfactant.

In at least one embodiment, the vesicle-forming surfactant is selected from the group consisting of nonionic, anionic, cationic, amphoteric surfactants, and mixtures thereof.

In at least one embodiment, the vesicle-forming surfactant comprises a non-ionic surfactant. In at least one embodiment, the non-ionic surfactant is selected from the group consisting of polyoxyethylene sorbitan esters, polyoxyethylene sorbitol esters, polyoxyalkylene fatty alcohol ethers, polyoxyalkylene fatty acid esters, alkoxylated glycerides, polyoxyethylene methyl glucoside ester, alkyl polyglucosides, EO-PO blockpolymers, or combinations of two or more thereof.

In at least one embodiment, the vesicle-forming surfactant comprises tricosaethylene glycol dodecyl ether.

In at least one embodiment, the vesicle-forming surfactant an anionic surfactant. In at least one embodiment, the anionic surfactant is selected from the group consisting of lactylic esters of fatty acids such as sodium lauroyl lactylate or sodium stearoyl lactylate, sulfonates of alkylbenzene-sulfonates, alkanesulfonates, olefinsulfonates, alkyl ether sulfate, alkyl sulfate, sulfo-succinates, alkyl phosphates, alkyl ether phosphates, protein fatty acid condensates, preferably collagen hydrolysates modified with fatty acid, amino acid-based surfactants, isethionates, taurides, acyl lactylates, neutralized fatty acids, or combinations of two or more thereof.

In at least one embodiment, the anionic, vesicle-forming surfactant is a salt of a lactylic ester of fatty acids.

In at least one embodiment, the vesicle-forming surfactant comprises a cationic surfactant. In at least one embodiment, the cationic surfactant is selected from the group consisting of esterquats, ditallow dimethyl ammonium chloride, $C_{12/14}$ alkyl dimethyl benzyl ammonium chloride, alkyl dimethyl benzil ammonium chloride, cetyl trimethyl ammonium chloride, stearyl trimethyl ammonium chloride, behenyl trimethyl ammonium chloride alkyl hydroxyethyl dimethyl ammonium chloride, distearyl dimethyl ammonium chloride, dihydrogenated tallow fatty alkyl dimethyl ammonium chloride, or combinations of two or more thereof.

In at least one embodiment, the vesicle-forming surfactant comprises an amphoteric surfactant. In at least one embodiment, the amphoteric surfactant is selected from the group consisting of alkyl amphoacetate, alkyl amidopropyl betaine, alkyl amidopropyl dimethylamine betaine, undecylenamidopropyl betaine, alkyl dimethyl amine oxide, and combinations thereof.

In at least one embodiment, the cosmetic gel comprises from 1 wt.% to 40 wt.% surfactant component. In at least one embodiment, the cosmetic gel comprises from 1.5 wt.% to 20 wt.%, preferably from 2 wt.% to 15 wt.%, more preferably from 2.5 wt.% to 12 wt.%, even more preferably from 3 wt.% to 10 wt.% surfactant component.

As stated, the surfactant component comprises a vesicle-forming surfactant. The surfactant component may consist of vesicle-forming surfactant. In at least one embodiment, the surfactant component further comprises one or more co-surfactants. A co-surfactant is a surfactant having a HLB value ≤6. Preferred co-surfactants have a HLB-value from 2 to 6.

In at least one embodiment, the co-surfactant is selected from the group consisting of sorbitan esters, citric esters, lactic esters, partial fatty acid glycerides, glycerol esters, polyglycerol esters, sorbitol esters, fatty alcohols, propylene glycol esters, methyl glucoside ester, alkyl polyglucosides, sugar esters, or combinations of two or more thereof.

In at least one embodiment, the co-surfactant is a blend of Glyceryl Stearate, Sodium Stearoyl Lactylate, Cetearyl Alcohol.

In at least one embodiment, the surfactant component comprises, as vesicle forming surfactant, a blend of an anionic surfactant and a non-ionic surfactant together with a co-surfactant or co-surfactant blend.

Advantageously, the weight ratio of oily phase to the surfactant component is from 80:0.1 to 80:30. In at least one embodiment, the weight ratio of oily phase to surfactant component is from 80:2 to 80:20. At ratios higher than those stated, the particles may coalesce, such that the particle size criterion of the invention is not met. At ratios lower than those stated, the particles gel form may become unstable.

A packing parameter PP can be assigned to a chemical species, for example to a surfactant or a triglyceride oil.

If several chemical species are present in certain concentrations to form a mixture of these species, a packing parameter of this mixture PPmixture can be calculated

$$PP_{mixture} = \left( \sum c_i * PP_i \right) / c_{total}$$

wherein

$PP_i$     is the packing parameter of the single species i,
$c_i$     is the concentration of the single species i in weight percent, and
$c_{total}$     is the total concentration of all i species in the mixture.

The lamellar vesicles according to the invention comprise vesicle-forming surfactant and triglyceride oil. Additionally, if present, the lamellar vesicles may comprise co-surfactant and/or oils, which are not triglyceride oils.

In one embodiment, the packing parameter is in the range from 0.5 to 1.5. If the packing parameter PP or $PP_{mixture}$ is less than 0.5, then micelles form. Micelles are present in a dynamic equilibrium and continually break down and re-form, so are unsuitable as storage media for other ingredients. As the packing parameter rises into the range of 0.3 - 0.5, rod-like micelles may form. Vesicles of the present invention are preferably formed when the packing parameter of the mixture of all participating surfactants and triglyceride oil is 0.5 or above. In the range 0.5 - 1, spherical vesicles form. If the shape of the vesicles is ellipsoid or disk like the packing parameter value may rise to values higher than 1.

The cosmetic gel comprises an aqueous phase which acts as a carrier for the multilamellar vesicles and as a carrier for water-soluble active ingredients.

In at least one embodiment, the cosmetic gel comprises at least 1 wt.% aqueous phase. In at least one embodiment, the cosmetic gel comprises from 2 wt.% to 30 wt.%, or from 2.5 wt.% to 20 wt.%, or from 3 wt.% to 17 wt.%, or from 3.5 wt.% to 16 wt.%, or from 4 wt.% to 15 wt.%, or from 4.5 wt.% to 14 wt.%, or from 5 wt.% to 13 wt.%, or from 5.5 wt.% to 12 wt.%, or from 6 wt.% to 11 wt.% aqueous phase, preferably wherein the aqueous phase is water.

The aqueous phase comprises a water-soluble polyhydric alcohol, which may assist in forming the vesicles.

In at least one embodiment, the cosmetic gel comprises from 1 wt.% to 15 wt.% and preferably from 3 wt.% to 10 wt.% of water soluble polyhydric alcohol.

In at least one embodiment, the water-soluble polyhydric alcohols are polyhydric alcohols having two or more hydroxyl groups in the molecule. In at least one embodiment, the water-soluble polyhydric alcohol is selected from the group consisting of: dihydric alcohols such as ethylene glycol, propylene glycol, trimethylene glycol, 1,2-butylene glycol, 1,3-butylene glycol, 1,4-butylene glycol, tetramethylene glycol, 2,3-butylene glycol, pentamethylene glycol, 2-butene-1,4-diol, hexylene glycol, octylene glycol; trihydric alcohols such as glycerine, trimethylol propane, 1,2,6-hexanetriol and the like; tetrahydric alcohols such as penthaerythritol; pentahydric alcohols such as xylytol, etc.; hexahydric alcohols such as sorbitol, mannitol; polyhydric alcohol polymers such as diethylene glycol, dipropylene glycol, polyethylene glycol, polypropylene glycol, tetraethylene glycol, diglycerine, polyethylene glycol, triglycerine, tetraglycerine, polyglycerine; dihydric alcohol alkyl ethers such as ethylene glycol monomethyl ether, ethylene glycol monoethyl ether, ethylene glycol monobutyl ether, ethylene glycol monophenyl ether, ethylene glycol monohexyl ether, ethylene glycol mono-2-methylhexyl ether, ethylene glycol isoamyl ether, ethylene glycol benzyl ether, ethylene glycol isopropyl ether, ethylene glycol dimethyl ether, ethylene glycol diethyl ether, ethylene glycol dibutyl ether; dihydric alcohol alkyl ethers such as diethylene glycol monomethyl ether, diethylene glycol monoethyl ether, diethylene glycol monobutyl ether, diethylene glycol dimethyl ether, diethylene glycol diethyl ether, diethylene glycol butyl ether, diethylene glycol methyl ethyl ether, triethylene glycol monomethyl ether, triethylene glycol monoethyl ether, propylene glycol monomethyl ether, propylene glycol monoethyl ether, propylene glycol monobutyl ether, propylene glycol isopropyl ether, dipropylene glycol methyl ether, dipropylene glycol ethyl ether, dipropylene glycol butyl ether; dihydric alcohol ether esters such as ethylene glycol monomethyl ether acetate, ethylene glycol monoethyl ether acetate, ethylene glycol monobutyl ether acetate, ethylene glycol monophenyl ether acetate, ethylene glycol diadipate, ethylene glycol disuccinate, diethylene glycol monoethyl ether acetate, diethylene glycol monobutyl ether acetate, propylene glycol monomethyl ether acetate, propylene glycol monoethyl ether acetate, propylene glycol monopropyl ether acetate, propylene glycol monophenyl ether acetate; glycerine monoalkyl ethers such as xyl alcohol, selachyl alcohol, batyl alcohol; sugar alcohols such as sorbitol, maltitol, maltotriose, mannitol, sucrose, erythritol, glucose, fructose, starch sugar, maltose, xylytose, starch sugar reduced alcohol, glysolid, tetrahydrofurfuryl alcohol, POE tetrahydrofurfuryl alcohol, POP butyl ether, POP POE butyl ether, tripolyoxypropylene glycerine ether, POP glycerine ether, POP glycerine ether phosphoric acid, POP POE pentanerythritol ether, and mixtures thereof. Advantageously, the polyhydric alcohol comprises 1,2-propylene glycol, 1,3-propylene glycol, glycerine or mixtures thereof.

The aqueous carrier is cosmetically acceptable. Advantageously, the aqueous carrier comprises water is useful for economic reasons and because it is cosmetically acceptable.

Optionally the cosmetic gel comprises water-miscible or water-soluble solvents such as lower alkyl alcohols. In at least one embodiment, the cosmetic gel comprises $C_1$-$C_5$ alkyl monohydric alcohols, preferably $C_2$-$C_3$ alkyl alcohols. The alcohols which may be present are in particular lower monohydric alcohols having 1 to 4 carbon atoms customarily used for cosmetic purposes, such as preferably ethanol and isopropanol.

In at least one embodiment, the cosmetic gel comprises a solvent comprises ethanol.

Natural solvents can also be used. In at least one embodiment, the cosmetic gel comprises a solvent selected from the group consisting of plant oil, honey, plant-derived sugar compositions, and mixtures thereof.

In at least one embodiment, the cosmetic gel comprises a polymeric amphiphilic component. As used herein, a component is "amphiphilic" if it has a log P value of 1 or above.

The amphiphilic character of a component may be determined by its partition coefficient between octanol and water. The octanol-water partition coefficient (log P) is a measure of the distribution of a substance between the aqueous and the organic octanol phase and is defined as follows

$$\log P_{\text{oct/wat}} = \log \left( \frac{[\text{solute}]_{\text{octanol}}^{\text{un-ionized}}}{[\text{solute}]_{\text{water}}^{\text{un-ionized}}} \right)$$

Examples of calculated and measured log P values are found in A. Leo, C. Hansch, D. Elkins, Chemical Reviews, Volume 71, no. 6, (1971).

Such polymeric amphiphilic components may assist in forming the lipid double layers of the vesicles.

In at least one embodiment, the additional polymeric amphiphilic component are selected from the group consisting of polyvinyl acetate, polyvinyl pyrrolidone, polyvinyl alcohols, vinyl pyrrolidone (VP)/hexadecene copolymer, VP/eicosene copolymer or silicone oils and their derivatives.

In at least one embodiment, the cosmetic gel comprises additives common in cosmetology, pharmacy, and dermatology, which are hereinafter called auxiliaries.

Exemplary auxiliaries include cationic polymers, stabilizers, care additives, preservatives, antifungal agents, astringent agents, deodorizing agents, sunscreens, anti-oxidants, dyes, pigments, other particulates, such as pearlescent materials, skin or hair active agents, such as water soluble vitamins and their derivatives and water soluble amino acids and their salts and/or derivatives, viscosity modifiers, nonvolatile solvents or diluents (both water soluble and insoluble), thickeners, foam boosters, pediculocides, pH-adjusting agents, chelants, proteins, skin active agents, caffeine, minoxidil, and combinations thereof.

In at least one embodiment, the cosmetic gel may comprise, as auxiliaries, from 0 wt.% to 5 wt.% vitamins and amino acids, by total weight of the formulation.

In at least one embodiment, the cosmetic gel comprises a cationic polymer auxiliary. Suitable cationic polymers include those known under the INCI designation "Polyquaternium", especially Polyquaternium-31, Polyquaternium-16, Polyquaternium-24, Polyquaternium-7, Polyquaternium-22, Polyquaternium-39, Polyquaternium-28, Polyquaternium-2, Polyquaternium-10, Polyquaternium-11, and also Polyquaternium 37 & mineral oil & PPG trideceth (Salcare SC95), PVP-dimethylaminoethyl methacrylate copolymer, guar-hydroxypropyltriammonium chlorides, and also calcium alginate and ammonium alginate. It is additionally possible to employ cationic cellulose derivatives; cationic starch; copolymers of diallylammonium salts and acrylamides; quaternized vinylpyrrolidone/vinylimidazole polymers; condensation products of polyglycols and amines; quaternized collagen polypeptides; quaternized wheat polypeptides; polyethyleneimines; cationic silicone polymers, such as amidomethicones, for example; copolymers of adipic acid and dimethylaminohydroxypropyldiethylenetriamine; polyaminopolyamide and cationic chitin derivatives, such as chitosan, for example.

In at least one embodiment, the cosmetic gel comprises a stabilizer auxiliary. Suitable stabilizers include metal salts of fatty acids, such as magnesium, aluminum and/or zinc stearate.

In at least one embodiment, the cosmetic gel comprises a care additive auxiliary. Suitable care additives include conventional ceramides, pseudoceramides, fatty acid N-alkylpolyhydroxyalkyl amides, cholesterol, cholesterol fatty acid esters, fatty acids, cerebrosides, phospholipids and panthenol.

In at least one embodiment, the cosmetic gel comprises, as auxiliary, a preservative or preservative system. Suitable preservatives include benzyl alcohol, piroctone olamine, phenoxyethanol, parabens, pentanediol, benzoic acid/sodium benzoate, sorbic acid/potassium sorbate, and other organic acids used to provide antimicrobial protection. Preservation boosting ingredients include anisic acid, lactic acid, sorbitan caprylate, ethylhexylglycerin, caprylyl glycol, octanediol, and similar substances.

In at least one embodiment, the cosmetic gel comprises 0.01 to 5 wt.%, particularly preferably from 0.05 wt.% to 1 wt.% of at least one preservative. Suitable preservatives include the substances listed in the International Cosmetic Ingredient Dictionary and Handbook, 9th Edition with the function "preservatives". In at least one embodiment, the preservative is selected from the group consisting of phenoxyethanol, benzyl paraben, butyl paraben, ethyl paraben, isobutyl paraben, isopropyl paraben, methyl paraben, propyl paraben, iodopropynyl butylcarbamate, methyldibromoglutaronitrile, DMDM hydantoin and combinations thereof. In at least one embodiment, the formulation comprises a preservative selected from the group consisting of cetyltrimethyl ammoniumchloride, cetylpyridinium chloride, benzethonium chloride, diisobutylethoxyethyldimethyl benzylammoniumchloride, sodium N-lauryl sarcosinate, sodium-N-palmethylsarcosinate, Lauroylsarcosine, N-myristoylglycine, potassium-N-laurylsarcosine, trimethylammoniumchloride, sodium aluminium chlorohydroxylactate, triethylcitrate, tricetylmethylammoniumchloride, 2,4,4'-trichloro-2'-hydroxydiphenylether (Triclosan), phenoxyethanol, 1,5-pentandiol, 1,6-hexandiol, 3,4,4'-trichlorocarbanilide (Triclocarban), diaminoalkylamide, L-lysine hexadecylamide, heavy metal citrate salts, salicylate, piroctose, zinc salts, pyrithione and its heavy metal salts, zinc pyrithione, zinc phenol sulfate, farnesol, ketoconazol, oxiconazol, bifonazole, butoconazole, cloconazole, clotrimazole, econazole, enilconazole, fenticonazole, isoconazole, miconazole, sulconazole, tioconazole, fluconazole, itraconazole, terconazole, naftifine, terbinafine, selenium disulfide, Octopirox®, methylchloroisothiazolinone, methylisothiazolinone, methyldibromo glutaronitrile, AgCl, chloroxylenol, sodium salts of diethylhexylsulfosuccinate, sodiumbenzoate, phenoxyethanol, benzylalkohol, phenoxyisopropanol, paraben, such as butyl-, ethyl-, methyl- und propylparaben, and their salts, pentandiol, 1,2-octanediol, ethylhexylglycerinw, benzylalcohol, sorbic acid, benzoic acid, lactic acid, imidazolidinyl urea, diazolidinyl urea, dimethylol dimethyl hydantoin (DMDMH), sodium salts of hydroxymethyl glycinate, hydroxyethylglycine of sorbic acid and combinations thereof. In at least one embodiment, the preservative is selected from the group consisting of phenoxyethanol, benzyl paraben, butyl paraben, ethyl paraben, isobutyl paraben, isopropyl paraben, methyl paraben, propyl paraben, iodopropynyl butylcarbamate, methyldibromoglutaronitrile, DMDM hydantoin and combinations thereof. In at least one embodiment, the formulation is substantially free of parabens.

In at least one embodiment, the cosmetic gel comprises an anti-fungal auxiliary. In at least one embodiment, the anti-fungal substance is selected from the group consisting of ketoconazole, oxiconazole, bifonazole, butoconazole, cloconazole, clotrimazole, econazole, enilconazole, fenticonazole, isoconazole, miconazole, sulconazole, tioconazole, fluconazole, itraconazole, terconazole, naftifine and terbinafine, zinc pyrithione, octopirox, and combinations thereof. In at least one embodiment, the formulation comprises a total amount of anti-fungal substance in the formulation of from 0.1 wt.% to 1 wt.%. In at least one embodiment, the formulation comprises a pyridinethione anti-dandruff particulates, for example 1-hydroxy-2-pyridinethione salts, are highly preferred particulate anti-dandruff agents. The concentration of pyridinethione antidandruff particulate may ranges from 0.1% to 4%, by weight of the cosmetic gel, preferably from 0.1 % to 3%, more preferably from 0.3% to 2%. Preferred pyridinethione salts include those formed from heavy metals such as zinc, tin, cadmium, magnesium, aluminum and zirconium, preferably zinc, more preferably the zinc salt of 1-hydroxy-2-pyridinethione (known as "zinc pyridinethione" or "ZPT"), more preferably 1-hydroxy-2-pyridinethione salts in platelet particle form. Salts formed from other cations, such as sodium, may also be suitable. Pyridinethione anti-dandruff agents are described, for example, in U.S. Pat. No. 2,809,971; U.S. Pat. No. 3,236,733; U.S. Pat. No. 3,753,196; U.S. Pat. No. 3,761,418; U.S. Pat. No. 4,345,080; U.S. Pat. No. 4,323,683; U.S. Pat. No. 4,379,753; and U.S. Pat. No. 4,470,982. It is contemplated that when ZPT is used as the anti-dandruff particulate in the formulations herein, that the growth or regrowth of hair may be stimulated or regulated, or both, or that hair loss may be reduced or inhibited, or that hair may appear thicker or fuller.

In at least one embodiment, the cosmetic gel comprises an astringent auxiliary. In at least one embodiment, the astringent is selected from the group consisting of magnesium oxide, aluminium oxide, titanium dioxide, zirconium dioxide, zinc oxide, oxide hydrates, aluminium oxide hydrate (boehmite) and hydroxide, chlorohydrates of calcium, magnesium, aluminium, titanium, zirconium or zinc. In at least one embodiment, the cosmetic gel comprises from 0.001 wt.% to 10 wt.%, or from 0.01 wt.% to 9 wt.%, or from 0.05 wt.% to 8 wt.%, or from 0.1 wt.% to 5 wt.% astringent.

In at least one embodiment, the cosmetic gel comprises a deodorising agent auxiliary. In at least one embodiment, the deodorising agent is selected from the group consisting of allantoin, bisabolol, and combinations thereof. In at least one embodiment, the cosmetic gel comprises from 0.001 wt.% to 10 wt.%, or from 0.01 wt.% to 9 wt.%, or from 0.05 wt.% to 8 wt.%, or from 0.1 wt.% to 5 wt.% deodorising agent.

In at least one embodiment, the cosmetic gel comprises a sun protection agent and/or UV filter auxiliary. Suitable sun protection agents and UV filters are disclosed in WO2013/017262A1 (published on 7th Feb 2013), from page 32, line 11 to the end of page 33. In at least one embodiment, the sun protection agent and/or UV filter is selected from the group consisting of 4-amino benzoic acid, 3-(4'-trimethylammonium)-benzylide-boran-2-one-methylsulfate, camphor benzalkonium methosulfate, 3,3,5-trimethyl-cyclohexylsalicylate, 2-hydroxy-4-methoxybenzophenone, 2-phenylbenzimidazole-5-sulfonic acid and potassium-, sodium- und triethanolamine salts thereof, 3,3'-(1,4-phenylene dimethine)-bis-(7,7-dimethyl-2-oxobicyclo[2.2.1]-heptane-1-methane sulfonic acid) and its salts, 1-(4-tert.-butylphenyl)-3-(4-methoxyphenyl)propan-1,3-dion, 3-(4'-sulfo)-benzylidene-bornane-2-one its salts, 2-cyan-3,3-diphenyl-acrylic acid-(2-ethylhexylester), polymers of N-[2(and 4)-(2-oxoborn-3-ylidenmethyl)benzyl]-acrylamide, 4-methoxycinnamic acid-2-ethyl-hexylester, ethoxylated ethyl-4-amino-benzoate, 4-methoxycinnamic acid-isoamylester, 2,4,6-tris-[p-(2-ethylhexyloxycarbonyl)anilino]-1,3,5-triazine, 2-(2H-benzotriazole-2-yl)-4-methyl-6-(2-methyl-3-(1,3,3,3-tetramethyl-1-(trimethylsilyloxy)-disiloxanyl)-propyl)phenol, 4,4'-[(6-[4-((1,1-dimethylethyl)-aminocarbonyl)phenylamino]-1,3,5-triazin-2,4-yl)diimino]bis-(benzoic acid-2-ethylhexylester), 3-benzophenone, 4-benzophenone (acic), 3(4'-methylbenzyliden)-D,L-camphor, 3-benzylidene-camphor, salicylic acid-2-ethylhexylester, 4- dimethyl aminobenzic acid-2-ethylhexylester, hydroxy-4-methoxy-benzophenone-5 sulfonic acid and the sodium salt thereof, 4-isopropyl benzylsalicylate, N,N,N-trimethyl-4-(2-oxoborn-3-ylidenemethyl) anilium methyl sulfate, homosalate (INN), oxybenzone (INN), 2-phenylbenzimidazole-5-sulfonic acid and its sodium, potassium, and triethanolamine salts, octylmethoxy cinnamic acid, isopentyl-4-methoxy cinnamic acid, isoamyl-p-methoxy cinnamic acid, 2,4,6-trianilino-(p-carbo-2'-ethylhexyl-1'-oxy)-1,3,5-triazine (octyl triazone) phenol, 2,2(2H-benzotriazol-2-yl)-4-methyl-6-(2-methyl-3-(1,3,3,3-tetramethyl-1-(trimethylsilyl)oxy)-disiloxanyl)propyl(drometrizole trisiloxane) benzic acid, 4,4-((6-(((1,1-dimethylethyl)amino)carbonyl)phenyl)amino)-1,3,5-triazine-2,4-diyl)diimino)bis,bis(2-ethylhexyl)ester) benzoic acid, 4,4-((6-(((1,1-dimethylethyl)amino)-carbonyl)phenyl)amino)-1,3,5-triazine-2,4-diyl)diimino)bis,bis(2-ethylhexyl)ester), 3-(4'-methylbenzylidene)-D,L-camphor (4-methylbenzylidene camphor), benzylidene-camphor-sulfonic acid, octocrylene, polyacrylamidomethyl-benzylidene-camphor, 2-ethylhexyl salicylate (octyl salicylate), 4-dimethyl-aminobenzoeacidethyl-2-hexylester (octyl dimethyl PABA), PEG-25 PABA, 2 hydroxy-4-methoxybenzo-phenone-5-sulfonic acid (5-benzophenone) and the sodium salt thereof, 2,2'-methylene-bis-6-(2H-benzotriazol-2-yl)-4-(tetramethyl-butyl)-1,1,3,3-phenol, the sodium salt of 2-2'-bis-(1,4-phenylene)1H-benzimidazole-4,6-disulfonic acid, (1,3,5)-triazine-2,4-bis((4-(2-ethyl-hexyloxy)-2-hydroxy)-phenyl)-6-(4-methoxyphenyl), 2-ethylhexyl-2-cyano-3,3-diphenyl-2-propenoate, glyceryl octanoate, di-p-methoxy cinnamic acid, p-amino-benzoic acid and its ester, 4-tert-butyl-4'-methoxydibenzoylmethane, 4-(2-β-glucopyranoxy)propoxy-2-hydroxybenzophenone, octyl salicylate, methyl-2,5-diisopropyl cinnamic acid, cinoxate, dihydroxy-dimethoxybenzophenone, disodium salts of 2,2'-dihydroxy-4,4'-dimethoxy-5,5'-disulfobenzophenone, dihydroxybenzophenone, 1,3,4-dimethoxyphenyl-4,4-dimethyl-1,3-pentanedione, 2-ethylhexyl-dimethoxybenzyliden-dioxoimidazolidinpropionate, methylene-bis-benztriazolyl tetramethylbutylphenol, phenyldibenzimidazoltetrasulfonate, bis-ethylhexyloxyphenol-methoxyphenol-triazine, tetrahydroxybenzophenone, terephthalylidendicamphor-sulfonic acid, 2,4,6-tris[4,2-ethylhexyloxycarbonyl)anilino]-1,3,5-triazine, methyl-bis(trimethylsiloxy)silyl-isopentyl trimethoxy cinnamic acid, amyl-p-dimethylaminobenzoate, amyl-p-dimethylamino benzoate, 2-ethylhexyl-p-dimethylaminobenzoate, isopropyl-p-methoxy cinnamic acid/diisopropyl cinnamic acid ester, 2-ethylhexyl-p-methoxy cinnamic acid, 2-hydroxy-4-methoxy benzophenone, 2-hydroxy-4-methoxy-benzophenone-5-sulfonic acid and the trihydrate, 2-hydroxy-4-methoxybenzophenone-5-sulfonate sodium salt, phenyl-benzimidazole sulfonic acid, and combinations thereof. In at least one embodiment, the cosmetic gel comprises from 0.001 wt.% to 10 wt.%, preferably from 0.05 wt.% to 5 wt.%, even more preferably from 0.1 wt.% to 3 wt.%, most preferably from 0.05 wt.% to 1 wt.% sun protection agent and/or UV filter. In at least one embodiment, the cosmetic gel comprises a photoprotective substance in an amount of from 0.01 to 10 wt.%, or from 0.1 to 5 wt.%, particularly preferably from 0.2 to 2 wt.%. The photoprotective substances include, in particular, all of the photoprotective substances specified in EP 1 084 696. In a preferred embodiment, the photoprotective substance is selected from the group consisting of 2-ethylhexyl 4-methoxycinnamate, methyl methoxycinnamate, 2-hydroxy-4-methoxybenzophenone-5-sulfonic acid, polyethoxylated p-aminobenzoates, and combinations thereof.

In at least one embodiment, the cosmetic gel comprises an anti-oxidant auxiliary. In at least one embodiment, the anti-oxidant is selected from the group consisting of amino acids, peptides, sugars, imidazoles, carotinoids, carotenes, chlorogenic acid, lipoic acid, thiols, thiol glycosyl esters, thiol N-acetyl esters, thiol methyl esters, thiol ethyl esters, thiol propyl esters, thiol amyl esters, thiol butyl esters, thiol lauryl esters, thiol palmitoyl esters, thiol oleyl esters, thiol linoleyl esters, thiol cholesteryl esters, thiol glyceryl esters, dilaurylthiodipropionate, distearylthiodipropionate, thiodipropionic acid, metal chelators, hydroxy acids, fatty acids, folic acids, vitamin C, tocopherol, vitamin A, stilbenes, derivatives and combinations thereof. In at least one embodiment, the anti-oxidant is selected from the group consisting of glycine, histidine, tyrosine, tryptophan, urocaninic acid, D,L-carnosine, D-carnosine, L-carnosine, beta-carotene, alpha-carotene, lycopene, dihydrolipoic acid, aurothioglucose, propylthiouracil, thioredoxine, glutathione, cysteine, cystine, cystamine, buthioninsulfoximine, homocysteinsulfoximine, buthioninsulfone, penta-, hexa-, heptathioninsulfoximine, hydroxyfatty acids, palmitic acid, phytinic acid, lactoferrin, citric acid, lactic acid, malic acid, humic acid, bile acid, bilirubin, biliverdin, EDTA, EGTA, linoleic acid, linolenic acid, oleic acid, butylhydroxyanisol, trihydroxybutyrophenone, ubichinon, ubichinol, ascorbylpalmitate, Mg-ascorbylphosphate, ascorbylacetate, vitamin E acetate, vitamin A palmitate, carnosine, mannose, ZnO, $ZnSO_4$, selenium methionine, stilbenes, superoxide dismutase, and combinations thereof. In at least one embodiment, the antioxidant is selected from the group consisting of vitamin A, vitamin A derivatives, vitamin E, vitamin E derivatives, and combinations thereof. In at least one embodiment, the cosmetic gel comprises from 0.001 wt.% to 10 wt.%, preferably from 0.05 wt.% to 5 wt.%, even more preferably from 0.1 wt.% to 3 wt.%, most preferably from 0.05 wt.% to 1 wt.% antioxidant.

In at least one embodiment, the cosmetic gel comprises a dye or pigment auxiliary. In at least one embodiment, the cosmetic gel comprises at least one pigment. Suitable dyes and pigments are disclosed in WO2013/017262A1 in the table spanning pages 36 to 43. These may be colored pigments which impart color effects to the product mass or to hair, or they may be luster effect pigments which impart luster effects to the product mass or to the hair. The color or luster effects on the hair are preferably temporary, i.e. they last until the next hair wash and can be removed again by washing the hair with customary shampoos. In at least one embodiment, the cosmetic gel comprises a total amount of from 0.01 wt.% to 25 wt.%, preferably from 5 wt.% to 15 wt.% pigment. In at least one embodiment, the particle size of the pigment is from 1 micron to 200 micron, preferably from 3 micron to 150 micron, more preferably 10 micron to 100 micron. The pigments are colorants which are virtually insoluble in the application medium, and may be inorganic or organic. Inorganic-organic mixed pigments are also possible. Preference is given to inorganic pigments. The advantage of inorganic pigments is their excellent resistance to light, weather and temperature. The inorganic pigments may be of natural origin. In at least one embodiment, the inorganic pigment is selected from the group consisting of chalk, ochre, umber, green earth, burnt sienna, graphite, and combinations thereof. The pigments may be white pigments, such as, for example, titanium dioxide or zinc oxide, black pigments, such as, for example, iron oxide black, colored pigments, such as, for example, ultramarine or iron oxide red, lustre pigments, metal effect pigments, pearlescent pigments, and fluorescent or phosphorescent pigments, where preferably at least one pigment is a colored, nonwhite pigment. In at least one embodiment, the pigment is selected from the group consisting of metal oxides, hydroxides and oxide hydrates, mixed phase pigments, sulfur-containing silicates, metal sulfides, complex metal cyanides, metal sulfates, chromates and molybdates, and the metals themselves (bronze pigments), and combinations thereof. In at least one embodiment, the pigment is selected from the group consisting of titanium dioxide (CI 77891), black iron oxide (CI 77499), yellow iron oxide (CI 77492), red and brown iron oxide (CI 77491), manganese violet (CI 77742), ultramarine (sodium aluminum sulfosilicates, CI 77007, Pigment Blue 29), chromium oxide hydrate (CI 77289), Prussian blue (ferric ferrocyanide, CI 77510), carmine (cochineal), and combinations thereof. In at least one embodiment, the pigment is selected from the group consisting of pearlescent and colored pigments based on mica which are coated with a metal oxide or a metal oxychloride, such as titanium dioxide or bismuth oxychloride, and optionally further color-imparting substances, such as iron oxides, Prussian blue, ultramarine, carmine etc. and where the color can be determined by varying the layer thickness. Such pigments are sold, for example, under the trade names Rona®, Colorona®, Dichrona® and Timiron® by Merck, Germany. In at least one embodiment, the pigment is selected from the group consisting of organic pigments such as sepia, gamboge, bone charcoal, Cassel brown, indigo, chlorophyll and other plant pigments. In at least one embodiment, the pigment is selected from the group consisting of synthetic organic pigments such as azo pigments, anthraquinoids, indigoids, dioxazine, quinacridone, phthalocyanine, isoindolinone, perylene and perinone, metal complex, alkali blue and diketopyrrolopyrrole pigments.

In at least one embodiment, the cosmetic gel comprises from 0.01 wt.% to 10 wt.%, preferably from 0.05 wt.% to 5 wt.%, of at least one particulate auxiliary. Suitable substances are, for example, substances which are solid at room temperature (25°C) and are in the form of particles. In at least one embodiment, the particulate substance is selected from the group consisting of silica, silicates, aluminates, clay earths, mica, insoluble salts, in particular insoluble inorganic metal salts, metal oxides, e.g. titanium dioxide, minerals and insoluble polymer particles are suitable. The particles are present in the formulation in undissolved, preferably stably dispersed form, and, following application to the keratin substrate and evaporation of the solvent, can deposit on the substrate in solid form. A stable dispersion can be achieved by providing the formulation with a yield point which is large enough to prevent the solid particles from sinking. An adequate yield point can be established using suitable gel formers in a suitable amount. In at least one embodiment, the particulate substance is selected from the group consisting of silica (silica gel, silicon dioxide) and metal salts, in particular inorganic metal salts, where silica is particularly preferred. Metal salts are, for example, alkali metal or alkaline earth metal halides, such as sodium chloride or potassium chloride; alkali metal or alkaline earth metal sulfates, such as sodium sulfate or magnesium sulfate.

In a second aspect, the invention relates to a cosmetic product comprising a packaging having a reservoir containing the cosmetic gel of the first aspect of the invention and a dispensing opening in fluid communication with the reservoir for dispensing the cosmetic gel. The packaging may comprise a pump dispenser for pumping cosmetic gel from the reservoir to the dispensing opening. Alternatively, the packaging may be configured as an aerosol dispenser. Advantageously, the packaging may be configured to convert the cosmetic gel into a mousse.

In a third aspect, the invention relates to the use of the cosmetic gel of the first aspect for hair conditioning, hair styling, skin moisturising and/or improving the wash fastness of a hair colorant in hair fibres.

In a fourth aspect, the invention relates to process for making the cosmetic gel according to the first aspect of the invention, the process comprising:

a) Providing a first phase comprising aqueous phase and the surfactant component;
b) Providing a second phase comprising the oily phase comprising the triglyceride oil and, optionally a co-surfactant;
c) Heating the first phase to a temperature from 20°C to 60 °C;
d) Introducing the second phase into the first phase while heating to a temperature from 50°C to 90°C;
e) Mixing the combined phases;
f) Forming a cosmetic gel according to any of claims 1 to 12.

In at least one embodiment, the mechanical mixing is carried performed using an emulsification device for manufacture of nano-emulsions. An example of a suitable emulsification device which may provide a narrow particle size distribution according to the invention is disclosed in US20130201785A1 (Dahms et al.; Assignee OTC GMBH, Oberhausen; publication date August 8th 2013).

In at least one embodiment, the emulsification device is for continuous production of emulsions and/or dispersions and comprises:

I. at least one mixing apparatus comprising a rotationally symmetric chamber sealed airtight on all sides, at least one inlet line for introduction of free-flowing components, at least one outlet line for discharge of the mixed free-flowing components, a stirrer unit which ensures laminar flow and comprises stirrer elements secured on a stirrer shaft, the axis of rotation of which runs along the axis of symmetry of the chamber and the stirrer shaft of which is guided on at least one side, wherein the at least one inlet line is arranged upstream of or below the at least one outlet line, wherein the ratio between the distance between inlet and outlet lines and the diameter of the chamber is ≥ 2:1, wherein the ratio between the distance between inlet and outlet lines and the length of the stirrer arms of the stirrer elements is 3:1 to 50:1, and wherein the ratio of the diameter of the stirrer shaft, based on the internal diameter of the chamber, is 0.25 to 0.75 times the internal diameter of the chamber, such that the components introduced into the mixing apparatus via the at least one inlet line are stirred and continuously transported by means of a turbulent mixing area on the inlet side, in which the components are mixed turbulently by the shear forces exerted by the stirrer units, a downstream percolating mixing area in which the components are mixed further and the turbulent flow decreases, a laminar mixing area on the outlet side, in which a lyotropic, liquid-crystalline phase is established in the mixture of the components, in the direction of the outlet line,

II. at least one drive for the stirrer unit and

III. at least one conveying device per component or per component mixture.

In an embodiment of the fourth aspect of the invention, the process comprises:

i. providing an emulsification device having a first inlet line in fluid communication with a turbulent mixing zone and a second inlet line in fluid communication with the turbulent mixing zone, a laminar zone in fluid communication with the turbulent mixing zone and an outlet line in fluid communication with the laminar zone;
ii. feeding the first phase into the first inlet line;
iii. feeding the second phase into the second inlet line;
iv. introducing the second phase into the first phase in the turbulent mixing zone and subjecting the phases to turbulent mixing;
v. transporting the mixed phases from the turbulent mixing zone into the laminar zone in which laminar flow of the mixed phases is established and vesicles form; and
vi. discharging the mixed phases comprising vesicles from the laminar zone of the emulsification device via the outlet line.

In a preferred embodiment of the inventive process the vesicles formed in the emulsification device are further diluted with aqueous phase, especially with water.
This can be performed in a separate device by introducing the vesicles into water. The aqueous phase may consist of water, or may comprise water and electrolytes or water and polyols. The polyols may comprise propylene glycol, polyethylene glycol, glycerin, polyglycerin, sorbitol, isosorbide, dimethyl isosorbide or mixtures thereof.

EXAMPLES

The examples which follow are intended to illustrate the subject matter of the invention without restricting it thereto.

These examples were prepared as follows: a first phase (A) was prepared, comprising aqueous phase and the surfactant component. A second phase (B) was prepared, comprising the oily phase including the triglyceride oil and co-surfactant. The first phase was heated to a temperature from 20°C to 60 °C. The second phase was then introduced into the first phase while heating to a temperature from 50°C to 90°C and the phases were mixed. The cosmetic gel according to the invention was then formed. The formed cosmetic gel was then combined with additional aqueous phase (C).

The examples were manufactured using an emulsification device, as disclosed in US20130201785A1. The emulsification device has a first inlet line in fluid communication with a turbulent mixing zone and a second inlet line in fluid communication with the turbulent mixing zone, a laminar zone in fluid communication with the turbulent mixing zone and an outlet line in fluid communication with the laminar zone. The first phase was fed into the first inlet line, the second phase was fed into the second inlet line, the second phase was introduced into the first phase in the turbulent mixing zone and the phases were subjected to turbulent mixing. The mixed phases were then transported from the turbulent mixing zone into the laminar zone in which laminar flow of the mixed phases was established and vesicles formed. The mixed phases comprising vesicles from the laminar zone of the emulsification device were then discharged via the outlet line after which additional aqueous was admixed to increase the total amount of aqueous phase.

Table 1: Example cosmetic gels

| Phase | | Component | Description | Ex. A1 grams |
|---|---|---|---|---|
| A | | Sodium Lauryl Lactate | Anionic surfactant | 2 |
| A | | Tricosaethylene glycol dodecyl ether [1] | Non-ionic surfactant | 4 |
| A | | Water | Carrier | 4 |
| A | | Glycerine | Water-soluble, polyhydric alcohol | 8 |

| Phase | Component | Description | Ex B1 grams | Ex. B2 grams | Ex. B3 grams | Ex. B4 grams | Ex. B5 grams |
|---|---|---|---|---|---|---|---|
| B | Coconut oil | triglyceride oil | 72.5 | 72.5 | 72,5 | 72,5 | 72,5 |
| B | Glyceryl Stearate, Sodium Stearoyl Lactylate, Cetearyl Alcohol [2] | Co-surfactant blend | 4 | 3.5 | 3 | 2,5 | 2 |
| B | Blend of Glyceryl Oleate, Polyglyceryl-3-polyricinoleate and Olea Europaea Oil Unsaponifiables [4] | Co-surfactant blend | - | - | - | - | - |

KEY:
[1] = Brij® L23 (CAS Number 9002-92-0) from Croda International PLC;
[2] = Biobase® S from Tri-K(http://www.tri-k.com/product/biobase-s/); QSP = quantity sufficient for 100%;
[3] = Rotefan GTTC;
[4] = Plantasens® Natural Emulsifier HP30 from Clariant.

| | Example | Expt. 1 grams | Expt. 2 grams | Expt. 3 grams | Expt. 4 grams | Expt. 5 grams | Expt. 6 grams | Expt. 7 grams | Expt. 8 grams | Expt. 9 grams | Expt. 10 grams | Expt. 11 grams | Expt. 12 grams | Expt. 13 grams | Expt. 14 grams |
|---|---|---|---|---|---|---|---|---|---|---|---|---|---|---|---|
| Phase A | A1 | 18 | 17,5 | 17 | 16,5 | 16 | 15,5 | 15 | 14,5 | 14 | 13,5 | 13 | 11 | 12 | 11,5 |
| Phase B | B1 | 76,5 | 76,5 | 76,5 | 76,5 | 76,5 | 76,5 | 76,5 | 76,5 | 76,5 | 76,5 | 76,5 | 76,5 | 76,5 | 76,5 |
| Phase C | Water | 5,5 | 6 | 6,5 | 7 | 7,5 | 8 | 8,5 | 9 | 9,5 | 10 | 10,5 | 12,5 | 11,5 | 12 |
| | median particle size d(50) in microns | 0,317 | 0,322 | 0,347 | 0,316 | 0,341 | 0,328 | 0,347 | 0,347 | 0,381 | 0,379 | 0,368 | 0,526 | 0,398 | 0,4 |
| | Standard deviation | 0,06 | 0,06 | 0,065 | 0,065 | 0,063 | 0,06 | 0,065 | 0,064 | 0,074 | 0,077 | 0,071 | 0,135 | 0,08 | 0,08 |

| | | Example | Expt. 15 grams | Expt. 16 grams | Expt. 17 grams | Expt. 18 grams | Expt. 19 grams | Expt. 20 grams | Expt. 21 grams | Expt. 22 grams | Expt. 23 grams | Expt. 24 grams |
|---|---|---|---|---|---|---|---|---|---|---|---|---|
| Phase A | | A1 | 18 | 18 | 18 | 18 | 18 | 18 | 18 | 18 | 18 | 18 |
| Phase B | | B1 | 76,5 | 72 | 65 | 68 | 60 | 40 | - | - | - | - |
| | | B2 | - | - | - | - | - | - | 76 | - | - | - |
| | | B3 | - | - | - | - | - | - | - | 75.5 | - | - |
| | | B4 | - | - | - | - | - | - | - | - | 75 | - |
| | | B5 | - | - | - | - | - | - | - | - | - | 74.5 |
| Phase C | | Water | 5,5 | 6 | 6,5 | 7 | 7,5 | 8 | 6 | 6,5 | 7 | 7,5 |
| | | median particle size d(50) in microns | 0,317 | 0,303 | 0,299 | 0,277 | 0,259 | 0,198 | 0,313 | 0,343 | 0,357 | 0,367 |
| | | Standard deviation | 0,06 | 0,06 | 0,065 | 0,065 | 0,063 | 0,06 | 0,054 | 0,061 | 0,067 | 0,07 |

**Claims**

1.  A cosmetic gel comprising multilamellar vesicles dispersed in an aqueous phase, wherein the multilamellar vesicles have the shape of a rotational body comprising two or more concentric lipid double layers, the cosmetic gel comprising:

    a) a surfactant component, comprising at least one vesicle-forming surfactant, which is a surfactant having a HLB value of greater than 6;
    b) an oily phase comprising a triglyceride oil;
    c) an aqueous phase comprising a water-soluble polyhydric alcohol;

    and wherein the multilamellar vesicles have a median particle size d(50) from 50 to 800 nanometres as determined by laser diffraction analysis using the Mie scattering Theory Evaluation, and wherein the particle size distribution is Gaussian and the standard deviation is less than 30% of the median particle size d(50) and wherein the cosmetic gel comprises from 15 wt.% to 80 wt.% of triglyceride oil.

2.  The cosmetic gel of claim 1, comprising from 20 wt.% to 75 wt.% more preferably from 30 wt.% to 70 wt.% of triglyceride oil.

3.  The cosmetic gel of claim 1 or 2, wherein the triglyceride oil comprises coconut oil, palm kernel oil, palm stearin oil, cotton stearin oil, corn oil, soybean oil, rice oil, jojoba oil, babusscu oil, pumpkin oil, grapeseed oil, sesame oil, walnut oil, apricot oil, macadamia oil, avocado oil, sweet almond oil, lady's-smock oil, castor oil, olive oil, peanut oil, rapeseed oil, argan oil, abyssinian oil, mustard oil, sesame oil, sunflower oil, olive oil, linseed oil, neem oil, almond oil, gooseberry oil, jojoba oil, rapeseed oil, Jasmine oil, caprylic capric triglyceride or mixtures thereof.

4.  The cosmetic gel of any preceding claim, wherein the triglyceride oil comprises and preferably consists of coconut oil.

5.  The cosmetic gel of any preceding claim, wherein the oily phase additionally comprises oils which are not triglyceride oils, and the cosmetic gel comprises at least 35 wt.%, preferably from 38 wt.% to 80 wt.%, more preferably 40 wt.% to 75 wt.%, even more preferably 50 wt.% to 73 wt.% oily phase.

6.  The cosmetic gel of any preceding claim comprising from 1 wt.% to 40 wt.%, preferably from 1.5 wt.% to 20 wt.%, more preferably from 3 wt.% to 10 wt.% of vesicle-forming surfactant.

7.  The cosmetic gel of any preceding claim, wherein the surfactant component comprises one or more co-surfactants, wherein a co-surfactant is a surfactant having a HLB value ≤6 preferably from 2 to 6.

8.  The cosmetic gel of any preceding claim, wherein the weight ratio of oily phase to surfactant component is 80:0.1 to 80:30.

9.  The cosmetic gel of any preceding claim, comprising at least 1 wt.% and preferably from 2 wt.% to 30 wt.% aqueous phase.

10. The cosmetic gel according to any preceding claim, comprising from 1 wt.% to 15 wt.% and preferably from 3 wt.% to 10 wt.% of water soluble polyhydric alcohol.

11. The cosmetic gel according to any preceding claim, wherein the water soluble polyhydric alcohol comprises 1,2-propylene glycol, 1,3-propylene glycol, glycerine or mixtures thereof.

12. The cosmetic gel of any preceding claim, comprising a polymeric amphiphilic component selected from the group consisting of polyvinyl acetate, polyvinyl pyrrolidone, polyvinyl alcohols, vinyl pyrrolidone (VP)/hexadecene copolymer, VP/eicosene copolymer, silicone oils and their derivatives, and mixtures thereof.

13. The cosmetic gel of any preceding claim, wherein the cosmetic gel is a leave-on cosmetic composition for conditioning skin or keratinous material.

14. A cosmetic product comprising a packaging having a reservoir containing the cosmetic gel of any preceding claim and a dispensing opening in fluid communication with the reservoir for dispensing the cosmetic gel.

**15.** Use of the cosmetic gel of any one of claims 1 to 12 for skin conditioning or moisturization or for hair conditioning.

**16.** A process of making the cosmetic gel, comprising:

a) Providing a first phase comprising aqueous phase and the surfactant component;
b) Providing a second phase comprising the oily phase comprising the triglyceride oil and, optionally a co-surfactant;
c) Heating the first phase to a temperature from 20°C to 60°C;
d) Introducing the second phase into the first phase while heating to a temperature from 50°C to 90°C;
e) Mixing the combined phases;
f) Forming a cosmetic gel according to any of claims 1 to 8.

**Patentansprüche**

**1.** Kosmetisches Gel, umfassend multilamellare Vesikel, die in einer wässrigen Phase dispergiert sind, wobei die multilamellaren Vesikel die Form eines Rotationskörpers aufweisen, der zwei oder mehr konzentrische Lipiddoppelschichten umfasst, wobei das kosmetische Gel umfasst:

a) eine Tensidkomponente, die wenigstens ein vesikelbildendes Tensid umfasst, das ein Tensid mit einem HLB-Wert von höher als 6 ist;
b) eine ölige Phase, die ein Triglyceridöl umfasst;
c) eine wässrige Phase, die einen wasserlöslichen polyhydrischen Alkohol umfasst;

und wobei die multilamellaren Vesikel eine mediane Partikelgröße d(50) von 50 bis 800 Nanometer, wie bestimmt durch Laserdiffraktionsanalyse unter Verwendung der Mie-Streutheorie-Auswertung, aufweisen, und wobei die Partikelgrößenverteilung gaußsch ist und die Standardabweichung weniger als 30 % der medianen Partikelgröße d(50) beträgt und wobei das kosmetische Gel von 15 Gew.-% bis 80 Gew.-% Triglyceridöl umfasst.

**2.** Kosmetisches Gel gemäß Anspruch 1, umfassend von 20 Gew.-% bis 75 Gew.-%, bevorzugter von 30 Gew.-% bis 70 Gew.-%, Triglyceridöl.

**3.** Kosmetisches Gel gemäß Anspruch 1 oder 2, wobei das Triglyceridöl Kokosnussöl, Palmkernöl, Palmstearinöl, Baumwollstearinöl, Maisöl, Sojabohnenöl, Reisöl, Jojobaöl, Babusscuöl, Kürbisöl, Traubenkernöl, Sesamöl, Walnussöl, Aprikosenöl, Macadamiaöl, Avocadoöl, Süßmandelöl, Wiesenschaumkrautöl, Castoröl, Olivenöl, Erdnussöl, Rapsöl, Arganöl, Abessinisches Öl, Senföl, Sesamöl, Sonnenblumenöl, Olivenöl, Leinsamenöl, Neemöl, Mandelöl, Stachelbeeröl, Jojobaöl, Rapsöl, Jasminöl, Capryl-Caprin-Triglycerid oder Gemische davon umfasst.

**4.** Kosmetisches Gel gemäß einem der vorstehenden Ansprüche, wobei das Triglyceridöl Kokosnussöl umfasst und vorzugsweise daraus besteht.

**5.** Kosmetisches Gel gemäß einem der vorstehenden Ansprüche, wobei die ölige Phase zusätzlich Öle umfasst, die keine Triglyceridöle sind, und das kosmetische Gel wenigstens 35 Gew.-%, vorzugsweise von 38 Gew.-% bis 80 Gew.-%, bevorzugter 40 Gew.-% bis 75 Gew.-%, noch bevorzugter 50 Gew.-% bis 73 Gew.-%, ölige Phase umfasst.

**6.** Kosmetisches Gel gemäß einem der vorstehenden Ansprüche, umfassend von 1 Gew.-% bis 40 Gew.-%, vorzugsweise von 1,5 Gew.-% bis 20 Gew.-%, bevorzugter von 3 Gew.-% bis 10 Gew.-%, an vesikelbildendem Tensid.

**7.** Kosmetisches Gel gemäß einem der vorstehenden Ansprüche, wobei die Tensidkomponente ein oder mehrere Cotenside umfasst, wobei ein Cotensid ein Tensid ist, das einem HLB-Wert von $\leq$ 6, vorzugsweise von 2 bis 6, aufweist.

**8.** Kosmetisches Gel gemäß einem der vorstehenden Ansprüche, wobei das Gewichtsverhältnis von öliger Phase zu Tensidkomponente 80:0,1 bis 80:30 beträgt.

**9.** Kosmetisches Gel gemäß einem der vorstehenden Ansprüche, umfassend wenigstens 1 Gew.-% und vorzugsweise von 2 Gew.-% bis 30 Gew.-% an wässriger Phase.

**10.** Kosmetisches Gel gemäß einem der vorstehenden Ansprüche, umfassend von 1 Gew.-% bis 15 Gew.-% und vorzugsweise von 3 Gew.-% bis 10 Gew.-% an wasserlöslichem polyhydrischem Alkohol.

**11.** Kosmetisches Gel gemäß einem der vorstehenden Ansprüche, wobei der wasserlösliche polyhydrische Alkohol 1,2-Propylenglycol, 1,3-Propylenglycol, Glycerin oder Gemische davon umfasst.

**12.** Kosmetisches Gel gemäß einem der vorstehenden Ansprüche, umfassend eine polymere amphiphile Komponente ausgewählt aus der Gruppe bestehend aus Polyvinylacetat, Polyvinylpyrrolidon, Polyvinylalkoholen, Vinylpyrrolidon (VP)/Hexadecen-Copolymer, VP/Eicosen-Copolymer, Siliconölen und deren Derivaten und Gemischen davon.

**13.** Kosmetisches Gel gemäß einem der vorstehenden Ansprüche, wobei das kosmetische Gel eine kosmetische Leave-on-Zusammensetzung zur Konditionierung von Haut oder Keratinmaterial ist.

**14.** Kosmetisches Produkt, umfassend eine Verpackung, die einen Behälter, der das kosmetische Gel gemäß einem der vorstehenden Ansprüche enthält, und eine Ausgabeöffnung in Fluidverbindung mit dem Behälter zum Ausgeben des kosmetischen Gels aufweist.

**15.** Verwendung des kosmetischen Gels gemäß einem der Ansprüche 1 bis 12 zur Konditionierung oder Befeuchtung von Haut oder zur Konditionierung von Haar.

**16.** Verfahren zur Herstellung des kosmetischen Gels, umfassend:

a) Bereitstellen einer ersten Phase, die wässrige Phase und die Tensidkomponente umfasst;
b) Bereitstellen einer zweiten Phase, die die ölige Phase, die das Triglyceridöl umfasst, und gegebenenfalls ein Cotensid umfasst;
c) Erhitzen der ersten Phase auf eine Temperatur von 20 °C bis 60 °C;
d) Einführen der zweiten Phase in die erste Phase unter Erhitzen auf eine Temperatur von 50 °C bis 90 °C;
e) Mischen der kombinierten Phasen;
f) Bilden eines kosmetischen Gels gemäß einem der Ansprüche 1 bis 8.

**Revendications**

**1.** Gel cosmétique comprenant des vésicules multilamellaires dispersées dans une phase aqueuse, les vésicules multilamellaires possédant la forme d'un corps rotationnel comprenant deux couches doubles lipidiques concentriques ou plus, le gel cosmétique comprenant :

a) un composant de type tensioactif, comprenant au moins un tensioactif de formation de vésicules, qui est un tensioactif possédant une valeur HLB supérieure à 6 ;
b) une phase huileuse comprenant une huile de triglycéride ;
c) une phase aqueuse comprenant un alcool polyhydrique soluble dans l'eau ;

et les vésicules multilamellaires possédant une taille médiane de particule d(50) de 50 à 800 nanomètres telle que déterminée par une analyse de diffraction laser à l'aide de l'Évaluation Théorique de diffusion de Mie et la distribution de taille de particule étant gaussienne et l'écart type étant inférieur à 30 % de la taille médiane de particule d(50) et le gel cosmétique comprenant de 15 % en poids à 80 % en poids d'huile de triglycéride.

**2.** Gel cosmétique selon la revendication 1, comprenant de 20 % en poids à 75 % en poids, plus préférablement de 30 % en poids à 70 % en poids d'huile de triglycéride.

**3.** Gel cosmétique selon la revendication 1 ou 2, l'huile de triglycéride comprenant de l'huile de noix de coco, de l'huile de noix de palme, de l'huile de stéarine de palme, de l'huile de stéarine de coton, de l'huile de maïs, de l'huile de soja, de l'huile de riz, de l'huile de jojoba, de l'huile de babusscu, de l'huile de courge, de l'huile de pépins de raisin, de l'huile de sésame, de l'huile de noix, de l'huile d'abricot, de l'huile de macadamia, de l'huile d'avocat, de l'huile d'amande douce, de l'huile de cardamine des prés, de l'huile de ricin, de l'huile d'olive, de l'huile d'arachide, de l'huile de colza, de l'huile d'argan, de l'huile d'Abyssinie, de l'huile de moutarde, de l'huile de sésame, de l'huile de tournesol, de l'huile d'olive, de l'huile de lin, de l'huile de neem, de l'huile d'amande, de l'huile de groseille à maquereaux, de l'huile de jojoba, de l'huile de colza, de l'huile de Jasmin, un triglycéride caprylique caprique ou des mélanges correspondants.

4. Gel cosmétique selon une quelconque revendication précédente, l'huile de triglycéride comprenant et préférablement étant constituée d'huile de noix de coco.

5. Gel cosmétique selon une quelconque revendication précédente, la phase huileuse comprenant de plus des huiles qui ne sont pas des huiles de triglycéride, et le gel cosmétique comprenant au moins 35 % en poids, préférablement de 38 % en poids à 80 % en poids, plus préférablement 40 % en poids à 75 % en poids, encore plus préférablement 50 % en poids à 73 % en poids de phase huileuse.

6. Gel cosmétique selon une quelconque revendication précédente comprenant de 1 % en poids à 40 % en poids, préférablement de 1,5 % en poids à 20 % en poids, plus préférablement de 3 % en poids à 10 % en poids de tensioactif de formation de vésicules.

7. Gel cosmétique selon une quelconque revendication précédente, le composant de type tensioactif comprenant un ou plusieurs co-tensioactifs, un co-tensioactif étant un tensioactif possédant une valeur HLB $\leq$ 6, préférablement de 2 à 6.

8. Gel cosmétique selon une quelconque revendication précédente, le rapport en poids de phase huileuse sur composant de type tensioactif étant de 80:0,1 à 80:30.

9. Gel cosmétique selon une quelconque revendication précédente, comprenant au moins 1 % en poids et préférablement de 2 % en poids à 30 % en poids de phase aqueuse.

10. Gel cosmétique selon une quelconque revendication précédente, comprenant de 1 % en poids à 15 % en poids et préférablement de 3 % en poids à 10 % en poids d'alcool polyhydrique soluble dans l'eau.

11. Gel cosmétique selon une quelconque revendication précédente, l'alcool polyhydrique soluble dans l'eau comprenant du 1,2-propylèneglycol, du 1,3-propylèneglycol, de la glycérine ou des mélanges correspondants.

12. Gel cosmétique selon une quelconque revendication précédente, comprenant un composant amphiphile polymérique choisi dans le groupe constitué par un poly(acétate de vinyle), une polyvinylpyrrolidone, des poly(alcool vinylique), un copolymère de vinylpyrrolidone (VP)/hexadécène, un copolymère de VP/éicosène, des huiles de silicone et leurs dérivés, et des mélanges correspondants.

13. Gel cosmétique selon une quelconque revendication précédente, le gel cosmétique étant une composition cosmétique sans rinçage pour le conditionnement de la peau ou d'une matière kératinique.

14. Produit cosmétique comprenant un emballage possédant un réservoir contenant le gel cosmétique selon une quelconque revendication précédente et un orifice de distribution en communication fluidique avec le réservoir pour la distribution du gel cosmétique.

15. Utilisation du gel cosmétique selon l'une quelconque des revendications 1 à 12 pour le conditionnement ou l'hydratation de la peau ou pour le conditionnement des cheveux.

16. Procédé de fabrication du gel cosmétique, comprenant :

   a) la mise à disposition d'une première phase comprenant une phase aqueuse et le composant de type tensioactif ;
   b) la mise à disposition d'une deuxième phase comprenant la phase huileuse comprenant l'huile de triglycéride et, éventuellement un co-tensioactif ;
   c) le chauffage de la première phase à une température de 20 °C à 60 °C ;
   d) l'introduction de la deuxième phase dans la première phase tout en chauffant à une température de 50 °C à 90 °C ;
   e) le mélange des phases combinées ;
   f) la formation d'un gel cosmétique selon l'une quelconque des revendications 1 à 8.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 100712255
- US 2001047039 A1
- US 2809971 A
- US 3236733 A
- US 3753196 A
- US 3761418 A
- US 4345080 A
- US 4323683 A
- US 4379753 A
- US 4470982 A
- WO 2013017262 A1
- EP 1084696 A
- US 20130201785 A1, Dahms

**Non-patent literature cited in the description**

- *Journal of the Society of Cosmetic Chemists,* 1954, vol. 5 (4), 249-256
- Gas/Liquid and Liquid/Liquid Interfaces. Proceedings of 2nd Inter-national Congress Surface Activity. Butterworths, 1957, 426-438
- **A. LEO ; C. HANSCH ; D. ELKINS.** *Chemical Reviews,* 1971, vol. 71 (6
- International Cosmetic Ingredient Dictionary and Handbook